# EUROPÄISCHE PATENTSCHRIFT

(11) **EP 1 637 537 B1**
(45) Veröffentlichungstag und Bekanntmachung des Hinweises auf die Patenterteilung: **02.05.2007**
(21) Anmeldenummer: 05020152.4
(22) Anmeldetag: 15.09.2005
(51) Int. Cl.: C07F 9/655, C07F 9/09, A61K 31/14, A61K 31/343

(54) **Neue Arzneimittel**
New medicaments
Nouveaux medicaments

(30) Priorität: 17.09.2004 DE 102004045220
(43) Veröffentlichungstag der Anmeldung: 22.03.2006
(73) Patentinhaber: Riemser Arzneimittel AG, 17493 Greifswald - Insel Riems (DE)
(72) Erfinder: Braun, Dagmar, 17493 Greifswald-Insel Riems (DE)
(74) Vertreter: Vossius & Partner

(56) Entgegenhaltungen:
- DE-A1- 1 793 364
- BASSI ET AL: "The etiology and management of gagging: A review of the literature" JOURNAL OF PROSTHETIC DENTISTRY, XX, XX, Bd. 91, Nr. 5, Mai 2004 (2004-05), Seiten 459-467, XP005012514 ISSN: 0022-3913
- DICKINSON CM, FISKE J.: "A review of of gagging problems in dentistry: Aetiology and classification" DENT UPDATE, Bd. 32, Nr. 1, 2005, Seiten 26-32, XP009059113

## Beschreibung

Die vorliegende Erfindung betrifft die Verwendung von Methantheliniumbromid, sowie neue Methanthelinderivate und ―analoga, deren pharmazeutisch verträgliche Salze, und Arzneimittel die diese umfassen zur Hemmung des Würgereizes.

### Stand der Technik

Vagantin® welches Methantheliniumbromid als Wirkstoff enthält wird zur Zeit bei übermäßigem Schwitzen, Reizblase, Ulcus ventriculi, Ulcus duodeni, Gastritis, Reizmagen, verbunden mit Hyperacidität, bei vermehrten Bewegungsvorgängen im Magen- und Darmbereich, sowie bei Reizkolon verwendet.
Methantheliumbromid (Vagantin®, N,N-Diethyl-N-methyl-2-(xanthenylcarbonyloxy)ethylammoniumbromid) hat die folgende Formel

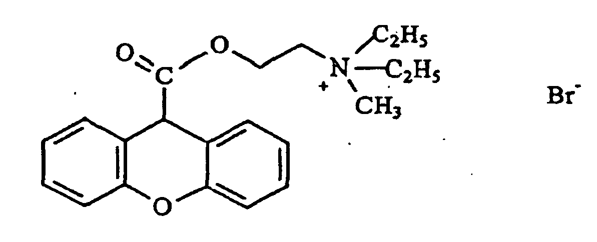

Methanthelinbromid entfaltet seine Wirkung vordergründig über die Blockade ganglionärer Zentren.
Ferner ist Methantheliniumbromid auf Grund seiner parasymphathikolytischen Funktionsweise und der antimuskarinergen Wirkungen in der Lage, eine Sekretionshemmung der Speicheldrüsen zu bewirken (Wilson, Edwin et al., Journal of Prosthetic Dentistry, Nov. 1984, Vol. 52, Nr. 5, S. 663-665, sowie Stephen, C.R. et al, Aenethesiology, 1956, 17, S. 303-313).

Ein (übermäßiger) Würgereflex stellt bei medizinischen Manipulationen im Mund-und Rachenraum ein großes Problem dar. Er wird durch den Druck verschiedener Behandlungsgeräte auf den Zungengrund und die hintere Rachenwand ausgelöst und wird durch Angst häufig verstärkt. Oft ist ein übermäßiger Würgereiz vor allem psychisch bedingt (Angst und Abwehrverhalten). Insbesondere Patienten mit einem leicht auslösbaren und/oder übermäßigen Würgereiz stellen für den behandelnden Zahnarzt/Arzt ein großes Problem dar, denn ein starker Würgereiz erschwert, wenn nicht sogar verunmöglicht viele Eingriffe beim Zahnarzt und in der Mundchirurgie.

Zur Bekämpfung bzw. Unterdrückung des Würgereizes bei Manipulationen im Mundbereich sind verschiedene Möglichkeiten bekannt.
So werden lokal anästhetisch wirkende Sprays auf die Mundschleimhaut gesprüht oder es werden hohe Dosen von Beruhigungsmitteln verabreicht.
Ein aufwendiges Verfahren ist auch die Akupressur bzw. die Akupunktur. Bestimmte Antiwürg - Punkte am Ohr werden durch Druck bzw. mit Akupunkturnadeln stimuliert. Dadurch werden chemische Prozesse in Gang gesetzt, die den Nervus Vagus beeinflussen.
Ähnlich positive Effekte sind auch über verschiedene Körperakupunkturpunkte zu erzielen.
(Deutsche Akademie für Akupunktur und Aurikulomedizin e. V., 1. Scriptum für die Aufbaustufe aller Akupunkturverfahren (Bahr, Dorfer, Schmid, Strittmatter/Suwanda/Leitler)
2. Akupunktur in der Zahn-, Mund- und Kieferheilkunde für Anfänger und mäßig Fortgeschrittene (Schmid, Nagel, Bahr).
Erfolge bei der Minderung des Würgereizes wurden auch mit der aufwendigen Methodik der Hypnose erreicht.
(Deutsche Gesellschaft für Zahnärztliche Hypnose e. V., 2003, Leuner, Hauscarl: Katathymen Bilderleben, Grundstufe Thieme Stuttgart 1982
Carnegie, Dale: Wie man Freunde gewinnt, Scherz-Verlag, Bern Fund München (1983))

Als weitere einfache Methoden den Würgereiz zu unterdrücken, wird die Massage des Nasenrückens, Gymnastik, Heimtraining mit zahnärztlichen Geräten (z. B. Abdrucklöffel) empfohlen.
Als aufwendige Methode ist eine gezielte Psychotherapie bekannt, um eventuell ursächlich traumatischen Erlebnisse zu überwinden.

Die vorstehende Darstellung des Standes der Technik macht deutlich, dass die empfohlenen Methoden und Verfahren die bei Problemen, wie sie bei medizinischen Manipulationen im Mund und Rachenraum entstehen, z. T. sehr aufwendig sind und auch eine längere gezielte Fachausbildung voraussetzen.

Aufgabe der Erfindung war es demnach neue Verbindungen und Arzneimittel zur Verhinderung und/oder Verminderung des Würgereizes, insbesondere in der zahnärztlichen und mundchirurgischen Praxis bereitzustellen.

### Beschreibung der Erfindung

Überraschenderweise hat man nun festgestellt, dass auch die erfindungsgemäßen Methantheliniumderivate und ―analoga und ihre pharmakologisch verträglichen Salze zur Verminderung oder Verhinderung des Würgereizes beitragen.

In einer ersten Ausführungsform betrifft somit die vorliegende Erfindung neue Methantheliniumderivate, -analoga der allgemeinen Formeln (ll) 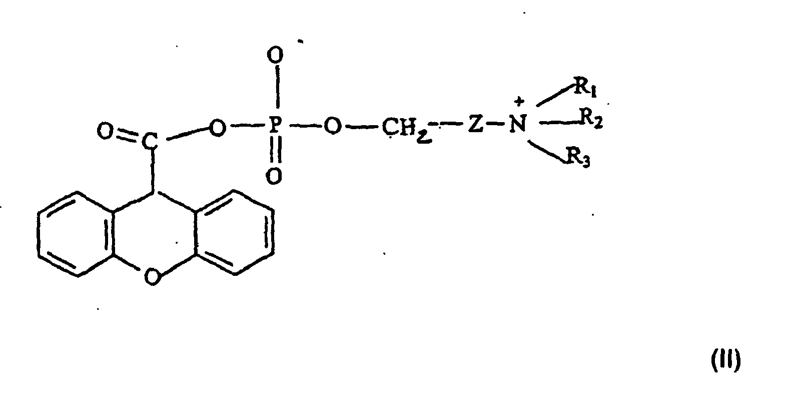 und deren pharmazeutisch verträglichen Salze,
wobei R₁, R₂ und R₃ gleich oder verschieden sind und ausgewählt sind aus einem Wasserstoffatom, einem C₁-C₅ -Alkylrest, insbesondere aus einer Methyl-, Ethyl- oder Propylgruppe;
n eine ganze Zahl ausgewählt aus 1, 2 und 3 ist;
Z ― (CH₂)ₘ- bedeutet, wobei m eine ganze Zahl aus 1, 2, 3, 4 und 5 ist.

Bevorzugt sind hierbei Verbindungen der Formel (II), wobei R₁=R₂=R₃.oder R₁, R₂ und R₃ verschieden sind.
Insbesondere bevorzugt sind die folgenden Verbindungen:
- 2-[(9-Xanthenylcarbonyloxy)phosphatidyl]ethylammonium
- N,N,N-Trimethyl-2-[(9-xanthenylcarbonyloxy)phosphatidyl]ethylammonium
- N,N,N-Triethyl-2-[(9-xanthenylcarbonyloxy)phosphatidyl]ethylammonium
- N,N,N-Tripropyl-2-[(9-xanthenylcarbonyloxy)phosphatidyl]ethylammonium
- N-Ethyl-N-methyl-N-propyl-2-[(9-xanthenylcarbonyloxy)phosphatidyl]ethylammonium
- 2-[(9-Xanthenylcarbonyloxy)phosphatidyl]butylammonium
- N,N,N-Trimethyl-2-[(9-xanthenylcarbonyloxy)phosphatidyl]butylammonium
- N,N,N-Triethyl-2-[(9-xanthenylcarbonyloxy)phosphatidyl]butylammonium
- N,N,N-Tripropyl-2-[(9-xanthenylcarbonyloxy)phosphatidyl]butylammonium
- N-Ethyl-N-methyl-N-propyl-2-[(9-xanthenylcarbonyloxy)phosphatidyl]butylammonium
- 2-[(9-Xanthenylcarbonyloxy)phosphatidyl]propylammonium
- N,N,N-Trimethyl-2-[(9-xanthenylcarbonyloxy)phosphatidyl]propylammonium
- N,N,N-Triethyl-2-[(9-xanthenylcarbonyloxy)phosphatidyl]propylammonium
- N,N,N-Tripropyl-2-[(9-xanthenylcarbonyloxy)phosphatidyl]propylammonium
- N-Ethyl-N-methyl-N-propyl-2-[(9-xanthenylcarbonyloxy)phosphatidyl]propylammonium
- 2-[(9-Xanthenylcarbonyloxy)phosphatidyl]pentylammonium
- N,N,N-Trimethyl-2-[(9-xanthenylcarbonyloxy)phosphatidyl]pentylammonium
- N,N,N-Triethyl-2-[(9-xanthenylcarbonyloxy)phosphatidyl]pentylammonium
- N,N,N-Tripropyl-2-[(9-xanthenylcarbonyloxy)phosphatidyl]pentylammonium
- N-Ethyl-N-methyl-N-propyl-2-[(9-xanthenylcarbonyloxy)phosphatidyl]pentylammonium

Diese Verbindungen können in der dem Fachmann an sich bekannter Weise hergestellt werden, wie zum Beispiel in HagerROM 2003. Hagers Handbuch der Drogen und Arzneistoffe, Blaschek, W.; Ebel, S.; Hackenthal, E.; Holzgrabe, U.; Keller, K.; Reichling, J. (Hrsg.) 3. Version, 2003, CD-ROM, ISBN: 3-540-14954-6 und Axel Kleemann, Jürgen Engel: Pharmaceutical Substances, Georg Thieme Verlag , Februar 2001 beschrieben.

Weiterhin betrifft die vorliegende Erfindung Arzneimittel welche die obengenannten neuen erfindungsgemäßen Verbindungen, gegebenenfalls zusammen mit einem oder mehreren verträglichen pharmazeutischen Exzipienten, umfassen.
Erfindungsgemäß zu verwendende Hilfsstoffe/Exzipienten umfassen hierbei Füllmittel wie. Maisstärke und andere Stärkearten, hochdisperses Siliziumdioxid, Lactose, Saccharose, Glucose, Mannit, Sorbit, Cellulose und Caliciumcarbonat.; Sprengmittel wie Stärkearten, wie Weizenstärke, Kartoffelstärke und Reisstärke; Bindemittel wie Polyvinylpyrrolidon, Polyvinylalkohol, Gelatine, Stärkearten, Tragant oder Gummi arabicum; Adsorbtionsmittel wie weißer Ton oder Siliziumdioxid; Überzugsmaterialien wie Macrogole Methylcellulose, Hydroxypropylmethylcellulose, Hydroxypropylcellulose, Ethylcellulose, Methylhydroxypropylcellulosephthalat, Celluloseacetatphthalat, Polyacrylate, und Carboxymethylcellulose-Natrium, Feuchthaltemittel wie Sorbit, oder Glycerin.; Lösungsmittel wie Kochsalzlösung, gereinigtes Wasser, Wasser zu Injektionszwecken, Ethanol, Macrogole, Isopropanol, Konservierungsmittel wie Benzylalkohol, Sorbinsäure, Benzoesäure oder para-Hydroxybenzoesäreester (PHB-Ester); Antioxidantien wie Ascorbinsäure, Butylhydroxyanisol oder Tocopherole.; Farbstoffe wie Titandioxid, Eisenoxide oder E100 (Kurkumin); Geschmacksstoffe wie Pfefferminz-, Zitronen- oder Orangengeschmack, Schmiermittel oder Gleitmittel wie Magnesiumstearat, Talkum, Macrogole.; Glättmittel wie Carnaubawachs und Fließregulierungsmittel wie hochdisperses Siliziumdioxid.

Ferner umfasst die vorliegende Erfindung die Verwendung von Methantheliniumbromid, Methantheliniumderivaten, -analoga und deren pharmazeutisch verträglichen Salzen der allgemeinen Formel (I) oder (II) 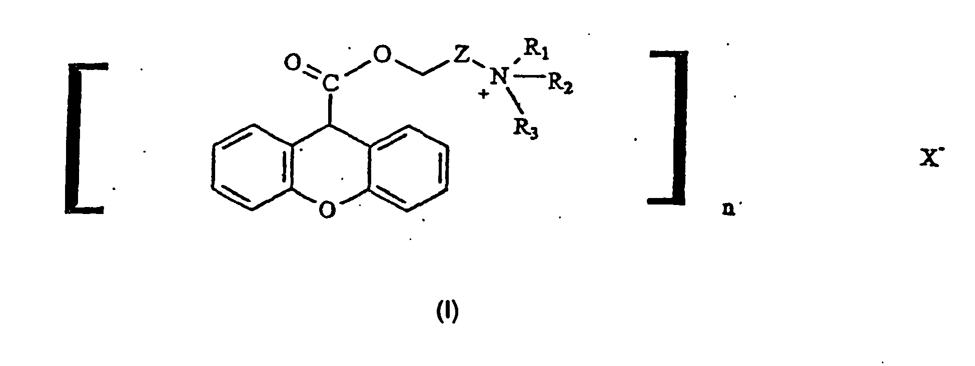 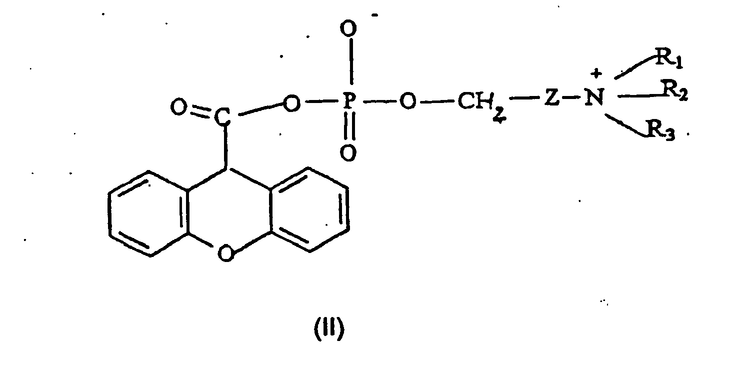 wobei R₁, R₂ und R₃ gleich oder verschieden sind und ausgewählt sind aus einem Wasserstoffatom, einer Methyl-, Ethyl- oder Propylgruppe;
n eine ganze Zahl ausgewählt aus 1, 2 und 3 ist;
Z ― (CH₂)ₘ- bedeutet, wobei m eine ganze Zahl aus 1, 2, 3, 4 und 5 ist,
und X⁻ F⁻, Cl⁻, Br⁻, I⁻, H₂PO₄⁻, CO₃²⁻, SO₄²⁻, HPO₄²⁻ oder PO₄³⁻ist, zur Verminderung oder Verhinderung des Würgereizes.

Die Verwendung von Verbindungen der Formel (I) oder (II), wobei R₁, R₂ und R₃ ausgewählt aus einem Wasserstoffatom und einer Methyl-, Ethyl-, oder Propylgruppe sind, ist bevorzugt.

Die Verwendung von Verbindungen der Formel (I) oder (II), wobei R₁=R₂=R₃ ist weiterhin bevorzugt.

Die Verwendung von Verbindungen der Formel (I) oder (II), wobei R₁, R₂ und R₃ verschieden sind, stellt eine weitere bevorzugte Ausführungsform dar.

Zusätzlich zu der Verwendung der obengenannten spezifischen Verbindungen der Formel (11) ist die Verwendung der folgenden Verbindungen der Formel (1)
- N,N,N-Trimethyl-2-(9-xanthenylcarbonyloxy)ethylammonium-Chlorid/Fluorid/Bromid/lodid//Dihydrogenphosphat
- N,N,N-Triethyl-2-(9-xanthenylcarbonyloxy)ethylammonium-Chlorid/Fluorid/Bromid/lodid//Dihydrogenphosphat
- N,N,N-Tripropyl-2-(9-xanthenylcarbonyloxy)ethylammonium-Chlorid/Fluorid/Bromid/lodid//Dihydrogenphosphat
- N-Ethyl-N-methyl-N-propyl-2-(9-xanthenylcarbonyloxy)ethylammonium-Chlorid/Fluorid/Bromid/lodid//Dihydrogenphosphat
- N,N,N-Trimethyl-2-(9-xanthenylcarbonyloxy)propylammonium-Chlorid/Fluorid/Bromid/lodid//Dihydrogenphosphat
- N,N,N-Triethyl-2-(9-xanthenylcarbonyloxy)propylammonium-Chlorid/Fluorid/Bromid/lodid//Dihydrogenphosphat
- N,N,N-Tripropyl-2-(9-xanthenylcarbonyloxy)propylammonium-Chlorid/Fluorid/Bromid/lodid//Dihydrogenphosphat
- N-Ethyl-N-methyl-N-propyl-2-(9-xanthenylcarbonyloxy)propylammonium-Chlorid/Fluorid/Bromid/lodid//Dihydrogenphosphat
- N,N,N-Trimethyl-2-(9-xanthenylcarbonyloxy)butylammonium-Chlorid/Fluorid/Bromid/lodid//Dihydrogenphosphat
- N,N,N-Triethyl-2-(9-xanthenylcarbonyloxy)butylammonium-Chlorid/Fluorid/Bromid/lodid//Dihydrogenphosphat
- N,N,N-Tripropyl-2-(9-xanthenylcarbonyloxy)butylammonium-Chlorid/Fluorid/Bromid/lodid//Dihydrogenphosphat
- N-Ethyl-N-methyl-N-propyl-2-(9-xanthenylcarbonyloxy)butylammonium-Chlorid/Fluorid/Bromid/lodid//Dihydrogenphosphat
- N,N,N-Trimethyl-2-(9-xanthenylcarbonyloxy)pentylammonium-Chlorid/Fluorid/Bromid/lodid//Dihydrogenphosphat
- N,N,N-Triethyl-2-(9-xanthenylcarbonyloxy)pentylammonium-Chlorid/Fluorid/Bromid/Iodid//Dihydrogenphosphat
- N,N,N-Tripropyl-2-(9-xanthenylcarbonyloxy)pentylammonium-Chlorid/Fluorid/Bromid/Iodid//Dihydrogenphosphat
- N-Ethyl-N-methyl-N-propyl-2-(9-xanthenylcarbonyloxy)pentylammonium-Chlorid/Fluorid/Bromid/lodid//Dihydrogenphosphat
- Di-[N,N,N-trimethyl-2-(9-xanthenylcarbonyloxy)ethylammonium]-Hydrogenphosphat/Carbonat/Sulfat
- Di-[N,N,N-triethyl-2-(9-xanthenylcarbonyloxy)ethylammonium]-Hydrogenphosphat/Carbonat/Sulfat
- Di-[N,N,N-tripropyl-2-(9-xanthenylcarbonyloxy)ethylammonium]-Hydrogenphosphat/Carbonat/Sulfat
- Di-[N-ethyl-N-methyl-N-propyl-2-(9-xanthenylcarbonyloxy)ethylammonium]-Hydrogenphosphat/Carbonat/Sulfat
- Di-[N,N,N-trimethyl-2-(9-xanthenylcarbonyloxy)propylammonium]-Hydrogenphosphat/Carbonat/Sulfat
- Di-[N,N,N-triethyl-2-(9-xanthenylcarbonyloxy)propylammonium]-Hydrogenphosphat/Carbonat/Sulfat
- Di-[N,N,N-tripropyl-2-(9-xanthenylcarbonyloxy)propylammonium]-Hydrogenphosphat/Carbonat/Sulfat
- Di-[N-ethyl-N-methyl-N-propyl-2-(9-xanthenylcarbonyloxy)propylammonium]-Hydrogenphosphat/Carbonat/Sulfat
- Di-[N,N,N-trimethyl-2-(9-xanthenylcarbonyloxy)butylammonium]-Hydrogenphosphat/Carbonat/Sulfat
- Di-[N,N,N-triethyl-2-(9-xanthenylcarbonyloxy)butylammonium]-Hydrogenphosphat/carbonat/Sulfat
- Di-[N,N,N-tripropyl-2-(9-xanthenylcarbonyloxy)butylammonium]-Hydrogenphosphat/Carbonat/Sulfat
- Di-[N-ethyl-N-methyl-N-propyl-2-(9-xanthenylcarbonyloxy)butylammonium]-Hydrogenphosphat/Carbonat/Sulfat
- Di-[N,N,N-trimethyl-2-(9-xanthenylcarbonyloxy)pentylammonium]-Hydrogenphosphat/Carbonat/Sulfat
- Di-[N,N,N-triethyl-2-(9-xanthenylcarbonyloxy)pentylammonium]-Hydrogenphosphat/carbonat/Sulfat
- Di-[N,N,N-tripropyl-2-(9-xanthenylcarbonyloxy)pentylammonium]-Hydrogenphosphat/Carbonat/Sulfat
- Di-[N-ethyl-N-methyl-N-propyl-2-(9-xanthenylcarbonyloxy)pentylammonium]-Hydrogenphosphat/Carbonat/Sulfat
- Tri-[N,N,N-trimethyl-2-(9-xanthenylcarbonyloxy)ethylammonium]-Phosphat
- Tri-[N,N,N-triethyl-2-(9-xanthenylcarbonyloxy)ethylammonium]-Phosphat
- Tri-[N,N,N-tripropyl-2-(9-xanthenylcarbonyloxy)ethylammonium]-Phosphat
- Tri-[N-ethyl-N-methyl-N-propyl-2-(9-xanthenylcarbonyloxy)ethylammonium]- Phosphat
- Tri-[N,N,N-trimethyl-2-(9-xanthenylcarbonyloxy)propylammonium]-Phosphat
- Tri-[N,N,N-triethyl-2-(9-xanthenylcarbonyloxy)propylammonium]-Phosphat
- Tri-[N,N,N-tripropyl-2-(9-xanthenylcarbonyloxy)propylammonium]-Phosphat
- Tri-[N-ethyl-N-methyl-N-propyl-2-(9-xanthenylcarbonyloxy)propylammonium]- Phosphat
- Tri-[N,N,N-trimethyl-2-(9-xanthenylcarbonyloxy)butylammonium]-Phosphat
- Tri-[N,N,N-triethyl-2-(9-xanthenylcarbonyloxy)butylammonium]-Phosphat
- Tri-[N,N,N-tripropyl-2-(9-xanthenylcarbonyloxy)butylammonium]-Phosphat
- Tri-[N-ethyl-N-methyl-N-propyl-2-(9-xanthenylcarbonyloxy)butylammonium]- Phosphat
- Tri-[N,N,N-trimethyl-2-(9-xanthenylcarbonyloxy)pentylammonium]-Phosphat
- Tri-[N,N,N-triethyl-2-(9-xanthenylcarbonyloxy)pentylammonium]-Phosphat
- Tri-[N,N,N-tripropyl-2-(9-xanthenylcarbonyloxy)pentylammonium]-Phosphat
- Tri-[N-ethyl-N-methyl-N-propyl-2-(9-xanthenylcarbonyloxy)pentylammonium]- Phosphat
bevorzugt, wobei die Verwendung von Methanthelinbromid besonders bevorzugt wird.

Erfindungsgemäß bevorzugt sind hierbei Arzneimittel zur oralen Applikation, insbesondere in der Form einer Lösung, einer Tablette oder in Form einer Kapsel. Ganz besonders bevorzugt sind Arzneimittel in Form insbesondere in Form eines Dragees oder einer Filmtablette,

Der Lösung können einige Tropfen Geschmackskorrigens, wie z.B. Pfefferminzgeschmack, hinzugefügt werden.
Als Lösungsmittel für eine orale Lösung können insbesondere Wasser, Kochsalzlösungen, Ethanol oder deren Mischungen dienen.
Bevorzugt sind Tablettenformulierungen die zusätzlich zum Wirkstoff Maisstärke, Polyvinylprrolidon, hochdisperses Siliziumdioxid, Lactosemonohydrat, Magnesiumsterarat, Hydroxypropylmethylcellulose, Talcum, Gummi arabicum
Calciumcarbonat, Titandioxid, Weißen Ton, Macogol 6000, Saccharose und E 100 umfassen.
Insbesondere ist die folgende Filmtabletten-Formulierung bevorzugt:
◆ 50,0 mg (erfindungsgemäßer Wirkstoff) z.B. Methanthelinbromid
◆ 30,0 mg (Maisstärke
◆ 18,75 mg (Polyvinylprrolidon
◆ 4,0 mg (hochdisperses Siliciumdioxid
◆ 146,25 mg (Lactosemonohydrat
◆ 1,0 mg vMagnesiumsterarat
◆ 1,0 mg (Hydroxypropylmethylzellulose
◆ 39,38 mg (Talcum
◆ 6,5 mg (Gummi arabicum
◆ 15,53 mg (Calciumcarbonat
◆ 7,73 mg vTitandioxid
◆ 6,58 mg (Weißer Ton
◆ 0,65 mg (Macogol 6000
◆ 61,63 mg (Sacharose
◆ 1,0 mg (E 100
◆ 0,1 mg (Carnauba Wachs

Bei Verwendung von Methantheliniumbromid entspricht die vorstehende Formulierung dem auf dem Markt befindlichen Vagantin®.

Besonders vorteilhaft gestaltet sich im Rahmen dieser Erfindung, dass neben der Beseitigung des Würgereflexes auch gleichzeitig die störende Speichelproduktion und das übermäßige Schwitzen durch Einwirkung auf die ganglionären Zentren im cholinergen System beseitigt wird.

Wie nachfolgend diskutiert und auch in den Figuren gezeigt, ist überraschenderweise die Verwendung einer Dosis von 100mg einer erfindungsgemäßen Verbindung ganz besonders wirkungsvoll und somit bevorzugt.

### Beispiel

Die Studie wurde an 12 männliche und weiblichen Probanden im Alter zwischen 18 und 45 Jahren durchgeführt. Der Wirkstoff (Methantheliniumbromid) wurden oral als Filmtablette oder als Lösung appliziert. Zu unterschiedlichen Zeiten nach der Dosierung wurden Messungen vorgenommen und Laborwerte zur Charakterisierung der Wirkung festgestellt.

Im Beispiel wurden 100 mg Wirkstoff als Filmtablette der folgenden Formulierung eingesetzt:
◆ 50,0 mg (Methantheliniumbromid
◆ 30,0 mg (Maisstärke
◆ 18,75 mg (Polyvinylprrolidon
◆ 4,0 mg (hochdisperses Siliziumdioxid
◆ 146,25 mg (Lactosemonohydrat
◆ 1,0 mg (Magnesiumsterarat
◆ 1,0 mg (Hydroxypropylmethylcellulose
◆ 39,38 mg (Talcum
◆ 6,5 mg (Gummi arabicum
◆ 15,53 mg ( Calciumcarbonat
◆ 7,73 mg (Titandioxid
◆ 6,58 mg (Weißer Ton
◆ 0,65 mg (Macogol 6000
◆ 61,63 mg (Saccharose
◆ 1,0 mg ( E 100
◆ 0,1 mg (Carnauba Wachs

Diese Formulierung entspricht dem auf dem Markt befindlichen Vagantin®.

Es ist auch möglich, eine reine Lösung von 100 mg Methantheliniumbromid in Wasser als Frischlösung herzustellen. Dabei empfiehlt es sich, den bitteren Geschmack des Wirkstoffes durch angenehme Geschmackskorrigentien zu überdecken. Hier bieten sich z. B. einige Spritzer eines Mundwassers mit Pfefferminzgeschmack an.

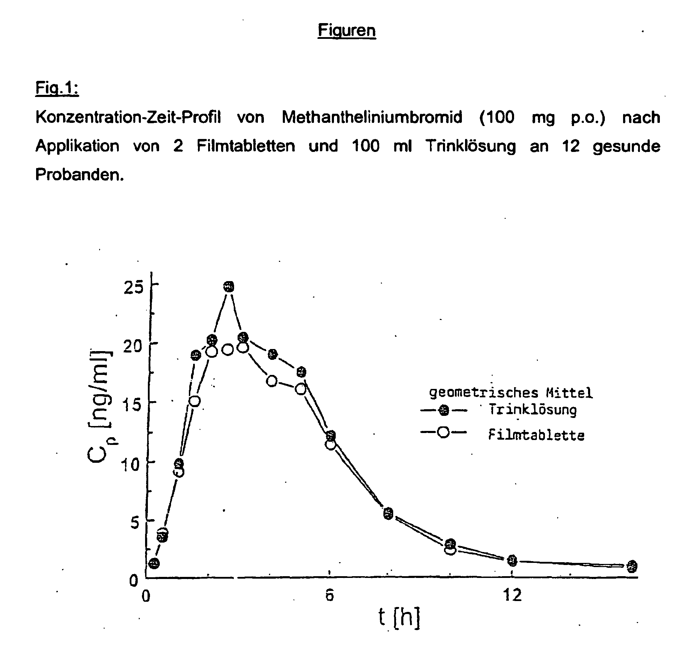

Der Wirkstoff Methantheliniumbromid in gelöster Form als Trinklösung zeigt eine etwas stärkere Reaktion als die Filmtablette. Ausdruck dafür ist das in der Figur 1 dargestellte Konzentrations-Zeit-Profil. Zu beachten ist eine deutliche individuelle Schwankungsbreite in der Serumkonzentration nach Zuführung beider Applikationsformen.

Methantheliniumbromid bzw. seine Analoga und Derivate werden nach Passage des oberen Verdauungstraktes und des Magens im Dünndarm resorbiert. Die Wirkung wird durch Proteine verzögert und gehemmt. Es ist daher angebracht, den Wirkstoff zur Erzielung einer optimalen Wirkung dem nüchternen Patienten zu applizieren, wenn die Wirkung sofort eintreten soll.

In einer klinischen Studie an 10 männlichen und weiblichen gesunden Probanden im Alter zwischen 20 und 60 Jahren wurde die Ausschaltung bzw. Verzögerung des Würgereizes und der Verminderung des Speichelflusses bei ärztlichen oder zahnärztlichen Manipulationen im Rachenraum geprüft.

Die obengenannte Filmtablettenformulierung bzw. die dort genannte Lösung von 100 mg Methantheliniumbromid in Wasser als Frischlösung wurde auch hier verwendet.

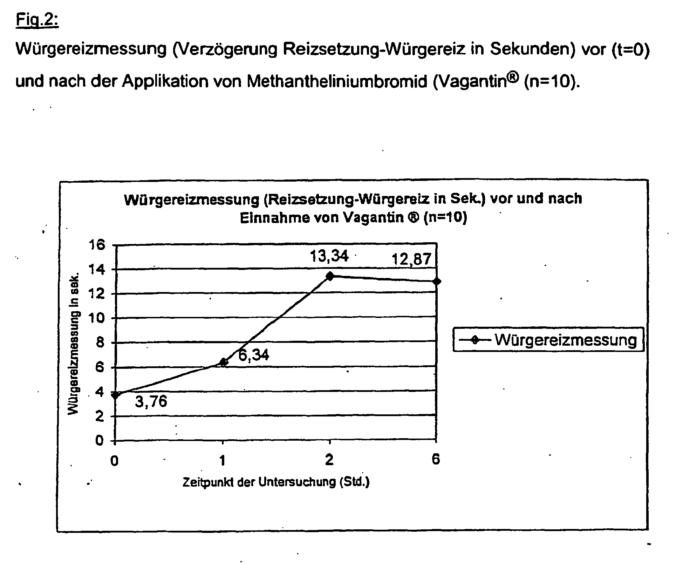

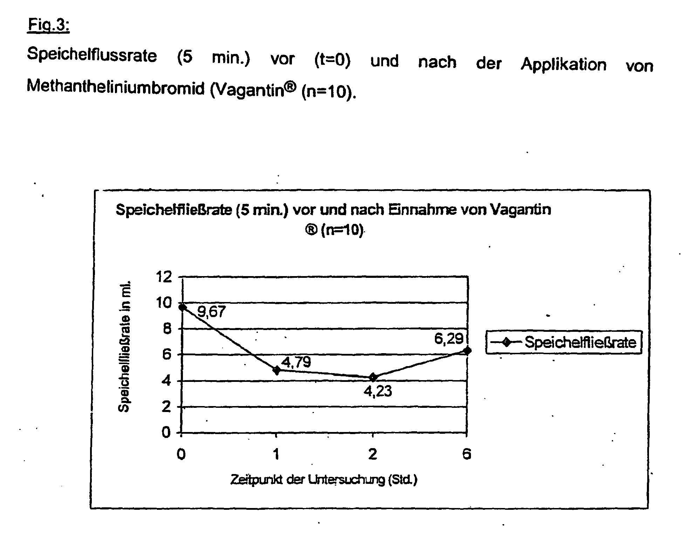

Nach Würgereizstimulus wurde die Würgereflexhemmung unmittelbar nach Applikation, sowie im Abstand von 1, 2 und 6 Stunden danach geprüft. Gleichzeitig wurde die Speichelflußrate vor und nach Einnahme von Methantheliniumbromid in Form von Vagantin® gemessen. Im Ergebnis auch dieser Untersuchungen wurde eine deutliche Verzögerung des Würgereizesreflexes und eine deutliche Reduzierung des Speichelflusses festgestellt. Die Ergebnisse sind in den Figuren. 2 und 3 dargestellt.

Durch die Sekretionshemmung der Speicheldrüsen und die Unterdrückung des Würgereizes wird für die ärztlichen bzw. zahnärztlichen Handlungen im Rachenbereich ein Operationsfeld geschaffen, das ein besseres, bequemeres und sicheres Arbeiten erlaubt. Die vordergründig peripheren bzw. ganglionären Angriffspunkte von Methantheliniumbromid schalten also zentralnervöse Reaktionen aus. Verbunden mit der bequemen oralen Applikation sind das bedeutende Vorteile, denn weil die Reaktionen auf die erfindungsgemäßen Wirkstoffe im nur Versorgungsgebiet der ganglionären Nervenknoten wirksam werden treten keine zentralnervösen Reaktionen auf, die den gesamten Organismus betreffen würden.

Figur 3 zeigt die Speichelsekretion in Abhängigkeit von der Methanthelinkonzentration im Plasma nach oraler Gabe von 100 mg Wirkstoff an gesunde Freiwillige.
Ferner kann von einem Methanthelinbromidgehalt im Plasma von etwa 5 ng/ml über einen Zweitraum von 8 Stunden ausgegangen werden (Figur 1).

Aus dem obengezeigten Beispiele wird deutlich, dass die vorliegende Erfindung wirksame Verbindungen zur Verminderung bzw. Verhinderung des Würgereizes, insbesondere in einer Dosierung von 100 mg bereitstellt.
Somit wird ein vor allem in der zahnäztlichen Praxis vielseitig einsetzbares Arzneimittel bereitstellt welches den bei Handhabungen im Mund- und Rachenbereich auftretenden Würgereiz unterbindet oder dämpft und auch den gleichzeitig auftretenden (vermehrten) Speichelfluß und übermäßiges Schwitzen verhindert oder verringert.

## Patentansprüche

1. Methantheliniumderivate, -analoga und deren pharmazeutisch verträglichen Salze der allgemeinen Formel (II) 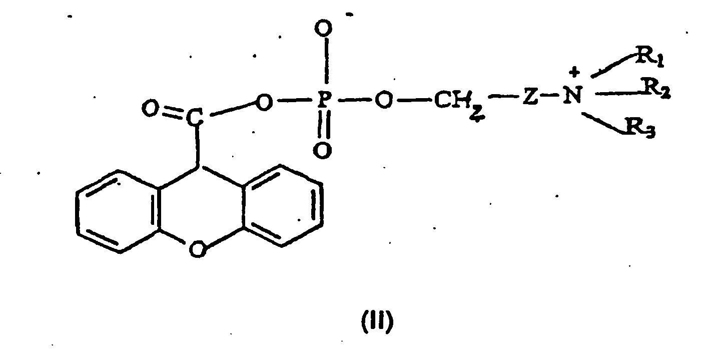
wobei R₁, R₂ und R₃ gleich oder verschieden sind und ausgewählt sind aus einem Wasserstoffatom, einer Methyl-, Ethyl- oder Propylgruppe;
Z - (CH₂)ₘ- bedeutet, wobei m eine ganze Zahl aus 1, 2, 3, 4 und 5 ist.

2. Verbindungen nach Anspruch 1, wobei R₁, R₂ und R₃ ausgewählt sind aus einem Wasserstoffatom, einer Methyl-, Ethyl-, oder Propylgruppe.

3. Verbindungen nach Anspruch 1 oder 2 wobei R₁=R₂=R₃.

4. Verbindungen nach Anspruch 1 oder 2, wobei R₁, R₂ und R₃ verschieden sind.

5. Arzneimittel umfassend eine Verbindung nach einem der Ansprüche 1 bis 4.

6. Verwendung von Methantheliniumbromid, Methantheliniumderivaten, analoga und deren pharmazeutisch verträglichen Salzen der allgemeinen Formel (I) oder (II) 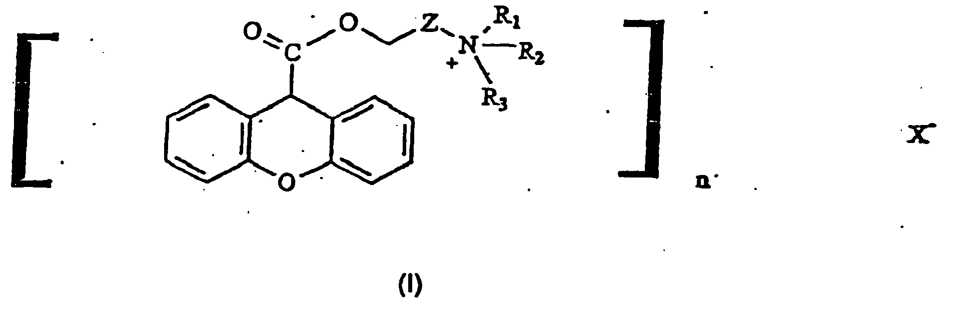 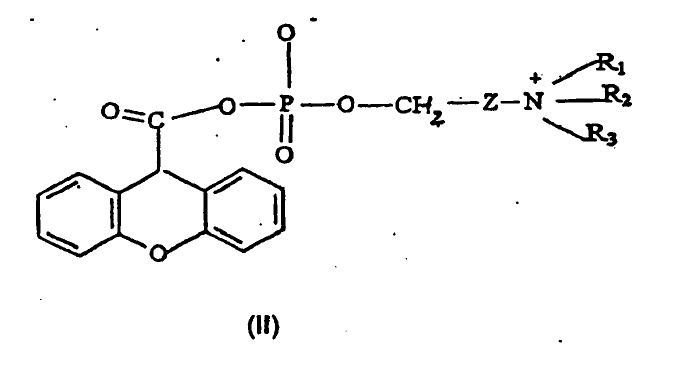
wobei R₁, R₂ und R₃ gleich oder verschieden sind und ausgewählt sind aus einem Wasserstoffatom, einer Methyl-, Ethyl- oder Propylgruppe; n eine ganze Zahl ausgewählt aus 1, 2 und 3 ist;
Z - (CH₂)ₘ- bedeutet, wobei m eine ganze Zahl aus 1, 2, 3, 4 und 5 ist, und X⁻ F⁻, Cl⁻, Br⁻, I⁻, H₂PO₄⁻, CO₃²⁻, SO₄²⁻, HPO₄²⁻ oder PO₄³⁻ist in der Herstellung eines Arzneimittels zur Verminderung oder Verhinderung des Würgereizes.

7. Verwendung nach Anspruch 6, wobei R₁, R₂ und R₃ ausgewählt sind aus einem Wasserstoffatom, einer Methyl-, Ethyl-, oder Propylgruppe.

8. Verwendung nach Anspruch 6 der Formel (I), wobei R₁=R₂=R₃.

9. Verwendung nach Anspruch 6 der Formel (II), wobei R₁=R₂=R₃.

10. Verwendung nach Anspruch 6 der Formel (I), wobei R₁, R₂ und R₃ verschieden sind.

11. Verwendung nach Anspruch 6 der Formel (II), wobei R₁, R₂ und R₃ verschieden sind.

12. Verwendung nach einem der Ansprüche 6 bis 11 bei ärztlichen oder zahnärztlichen Manipulationen im Mund- und Rachenraum.

13. Verwendung nach Anspruch 6 oder 12 zur oralen Applikation.

14. Verwendung nach einem der Ansprüche 6 bis 13, **dadurch gekennzeichnet, dass** 100 mg der Verbindung eingesetzt werden.

15. Verwendung nach einem der Ansprüche 6 bis 14, zur gleichzeitigen Verminderung oder Verhinderung der Trias von Speichelfluß, fokaler Hyperhidrose und Würgereiz als Komplex.

## Claims

1. Methanthelinium derivatives, analogs and pharmaceutical acceptable salts thereof, represented by the general formula (II) 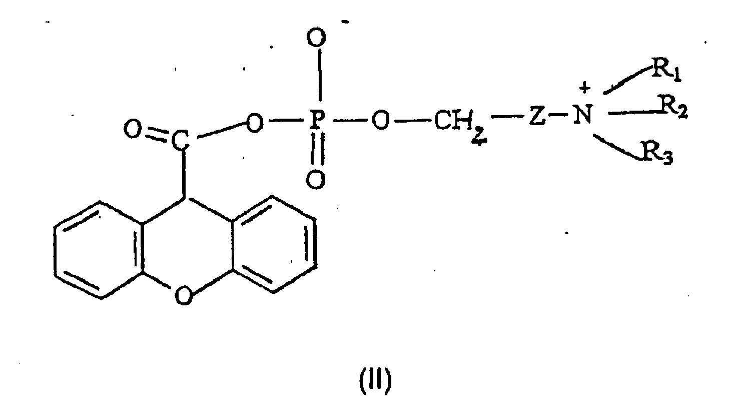
wherein R₁, R₂ and R₃ are the same or different and are selected from the group consisting of a hydrogen atom, a methyl, ethyl and propyl group;
Z is -(CH₂)ₘ-, wherein m is an integer of 1, 2, 3, 4 and 5.

2. The compounds according to claim 1, wherein R₁, R₂ and R₃ are selected from the group consisting of a hydrogen atom, a methyl, ethyl and propyl group.

3. The compounds according to claim 1 or 2, wherein R₁ = R₂ = R₃.

4. The compounds according to claim 1 or 2, wherein R₁, R₂ and R₃ are different.

5. A pharmaceutical composition comprising a compound according to any one of claims 1 to 4.

6. The use of methanthelinium bromide, methanthelinium derivatives, analogs and pharmaceutical acceptable salts thereof represented by the general formula (I) or (II) 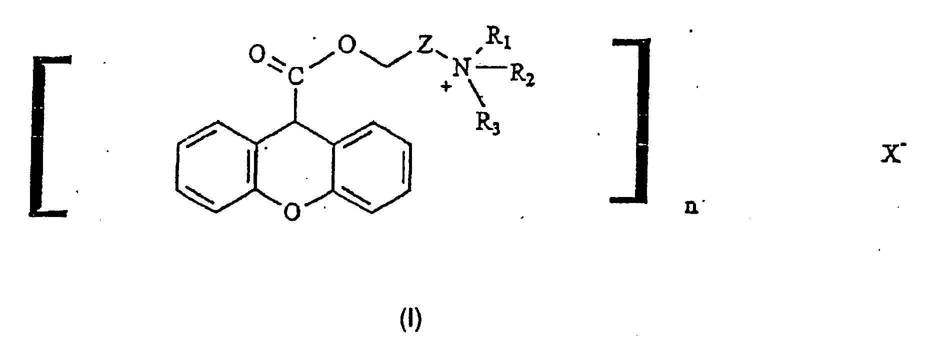 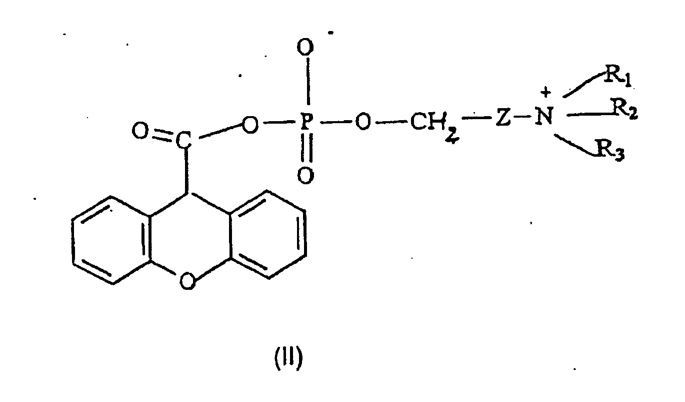
wherein R₁, R₂ and R₃ are the same or different and are selected from the group consisting of a hydrogen atom, a methyl, ethyl and propyl group;
n is an integer selected from 1, 2 and 3;
Z is -(CH₂)ₘ-, wherein m is an integer selected from 1, 2, 3, 4 and 5,
and X⁻ is F⁻, Cl⁻, Br⁻, I⁻, H₂PO₄⁻, CO₃²⁻, SO₄²⁻, HPO₄²⁻ or PO₄³⁻, in the manufacture of a pharmaceutical composition for the reduction or prevention of the gag reflex.

7. The use according to claim 6, wherein R₁, R₂ and R₃ are selected from the group consisting of a hydrogen atom, a methyl, ethyl and propyl group.
consisting of a hydrogen atom, a methyl, ethyl and propyl group.

8. The use according to claim 6 of formula (I), wherein R₁ = R₂ = R₃.

9. The use according to claim 6 of formula (II), wherein R₁ = R₂ = R₃.

10. The use according to claim 6 of formula (I), wherein R₁, R₂ and R₃ are different.

11. The use according to claim 6 of formula (II), wherein R₁, R₂ and R₃ are different.

12. The use according to any one of claims 6 to 11 during medical or dental manipulations in the oropharyngeal cavity.

13. The use according to claim 6 or 12 for oral application.

14. The use according to any one of claims 6 to 13, **characterized in that** 100 mg of the compound are used.

15. The use according to any one of claims 6 to 14, for the simultaneous reduction or prevention of the triad of sialism, focal hyperhidrosis and gag reflex as a complex.

## Revendications

1. Dérivés, analogues de méthanthélinium et leurs sels pharmaceutiquement acceptables de formule générale (II) : 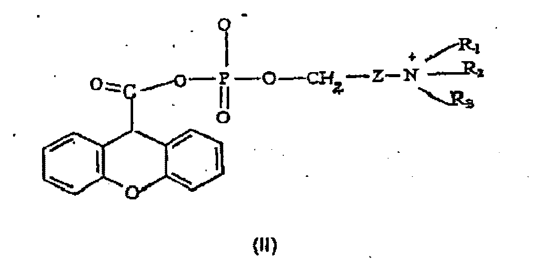
où R₁, R₂ et R₃, identiques ou différents, sont choisis parmi un atome d'hydrogène, un groupe méthyle, éthyle ou propyle ;
Z représente -(CH₂)ₘ-, où m est un nombre entier choisi parmi 1, 2, 3, 4 et 5.

2. Composés selon la revendication 1, où R₁, R₂ et R₃ sont choisis parmi un atome d'hydrogène et un groupe méthyle, éthyle ou propyle.

3. Composés selon la revendication 1 ou 2, où R₁ = R₂ = R₃.

4. Composés selon la revendication 1 ou 2, où R₁, R₂ et R₃ sont différents.

5. Médicament comprenant un composé selon l'une des revendications 1 à 4.

6. Utilisation de bromure de méthanthélinium, de dérivés, analogues de méthanthélinium et de leurs sels pharmaceutiquement acceptables, de formule générale (I) ou (II) : 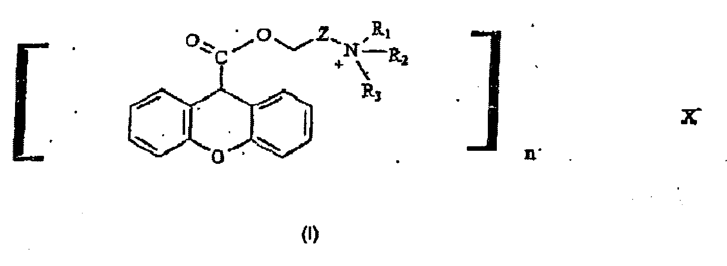 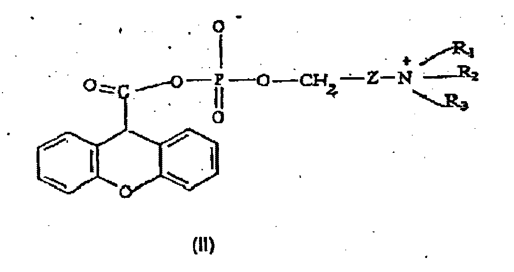
où R₁, R₂ et R₃, identiques ou différents, sont choisis parmi un atome d'hydrogène, un groupe méthyle, éthyle ou propyle ;
n est un nombre entier choisi parmi 1, 2 et 3 ;
Z représente -(CH₂)ₘ-, où m est un nombre entier choisi parmi 1, 2, 3, 4 et 5, et
X⁻ est F⁻, Cl⁻, Br⁻, I⁻, H₂PO₄⁻, CO₃²⁻, SO₄²⁻, HPO₄²⁻ ou PO₄³⁻, dans la préparation d'un médicament pour diminuer ou empêcher la nausée.

7. Utilisation selon la revendication 6, où R₁, R₂ et R₃ sont choisis parmi un atome d'hydrogène et un groupe méthyle, éthyle ou propyle.

8. Utilisation selon la revendication 6 de la formule (I), où R₁ = R₂ = R₃.

9. Utilisation selon la revendication 6 de la formule (II), où R₁ = R₂ = R₃.

10. Utilisation selon la revendication 6 de la formule (I), où R₁, R₂ et R₃ sont différents.

11. Utilisation selon la revendication 6 de la formule (II), où R₁, R₂ et R₃ sont différents.

12. Utilisation selon l'une des revendications 6 à 11, lors de manipulations médicales ou dentaires dans l'espace buccal et pharyngé.

13. Utilisation selon la revendication 6 ou 12, pour une administration orale.

14. Utilisation selon l'une des revendications 6 à 13, **caractérisée en ce que** l'on met en oeuvre 100 mg de composé.

15. Utilisation selon l'une des revendications 6 à 14, pour diminuer ou empêcher simultanément la triade de l'écoulement de salive, de l'hyperhidrose focale et de la nausée en tant que complexe.
